# EUROPEAN PATENT APPLICATION

(11) **EP 1 013 772 A1**
(43) Date of publication of application: **28.06.2000**
(21) Application number: 98403267.2
(22) Date of filing: 21.12.1998
(51) Int. Cl.: C12N 15/87, C12N 15/88, A61K 48/00, A61K 9/127

(54) **Method for preparing suspension of stable posiplexes**

(71) Applicant: TRANSGENE S.A., 67082 Strasbourg Cédex (FR)
(72) Inventor: Boussif, Otmane, 67000 Strasbourg (FR); Meyer, Olivier, 67000 Strasbourg (FR); Kolbe, Hanno V.J., 67204 Achenheim (FR)
(74) Representative: VOSSIUS & PARTNER

(57) **Abstract**

Described are stable posiplexes and to homogenous suspension of stable posiplexes that can be used to deliver nucleic acid to a cell for the purpose of providing a therapeutic molecule to the cells of an individual in need of such treatment. Furthermore, methods for the preparation of stable posiplexes and homogenous suspension thereof are provided.

## Description

The present invention relates to stable posiplexes and to homogenous suspension of stable posiplexes that can be used to deliver nucleic acid to a cell for the purpose of providing a therapeutic molecule to the cells of an individual in need of such treatment, and to methods for the preparation of an homogenous suspension of stable posiplexes.

Genetic diseases are due, in particular, to dysfunction in the expression of specific genes or to the expression of non-functional mutated polypeptides. Cystic fibrosis, for example, is regarded as the most frequently occurring of the lethal genetic diseases (1/2000) with a mean life expectancy of from 25 to 30 years, and is characterized by substantial thickening of the secretions derived from the mucous membranes, by chronic pulmonary attacks and by insufficiency of the exocrine pancreas. Said disease is associated with disturbances in the transport of electrolytes, in particular chloride, across the epithelial membrane, which disturbances are the consequence of mutations in the CFTR (cystic fibrosis transmembrane conductance regulator) gene (Rommens, Science 245 (1989), 1059-1066; Rosenfeld, Chest 109 (1996), 241-252). The therapeutic solution which appears to be most suitable for this type of disorder is that of transferring the gene encoding a functional CFTR polypeptide into the target cells in order to correct the observed cellular dysfunction. Within the context of this approach, also termed gene therapy, several authors indicate that the cells of the respiratory epithelium are a target of choice, especially as a result of its accessibility, in particular by means of intrapulmonary delivery, for example by instillation into the lungs. It has been shown that a level of expression of approximately 5 to 7% of that of the normal CFTR protein has to be obtained in the target cells for restoration of the electrolyte transport to be observed. Similarly, several publications describe the possibility of eliminating tumours or, failing that, of delaying their progress, by using the technique of transferring genes into the cancerous target cells. Several approaches have been considered, in particular transferring immunostimulatory genes (immunotherapy) which are able to induce or activate a cell-mediated immune response against the tumour; as examples of the therapeutic uses of such genes, mention may be made of the administration of genes which encode cytokines; transferring cytotoxic genes which confer toxicity on cells which express them, for example the thymidine kinase (tk) gene of type 1 herpes simplex virus (HSV-1); or transferring tumour suppressor genes, such as the retinoblastoma gene or the p53 gene, or polynucleotides which are able to inhibit the activity of an oncogene, such as antisense molecules or ribozymes which are able to degrade the specific messenger RNAs of the oncogenes.

Over the course of the last 30 years, a large number of tools have been developed for introducing various heterologous genes into cells, in particular mammalian cells. These different techniques can be divided into two categories. The first category relates to physical techniques such as microinjection, electroporation or particle bombardment which, although effective, are to a large extent limited to in vitro applications, the implementation of which is cumbersome. The second category involves techniques relating to molecular and cell biology, where the gene to be transferred is combined with a biological or synthetic vector which promotes the introduction of the said material. The vectors which are currently most effective are viral vectors, in particular adenoviral or retroviral vectors. The techniques which have been developed are based on the natural properties which these viruses possess for traversing cell membranes, evading degradation of their genetic material and enabling their genome to penetrate into the nucleus. These viruses have already been the subject of a large number of studies, and some of them are already employed experimentally as gene vectors in man with a view, for example, to vaccination, immunotherapy or therapy which is aimed at compensating for a genetic deficiency. Nevertheless, this viral approach suffers from a large number of limitations, in particular on account of the restricted cloning capacity within the viral genome, of the risks of infectious viral particles which have been produced being disseminated in the host organism and in the environment, of the risk of artefactual mutagenesis resulting from insertion in the host cell, in the case of retroviral vectors, and of the fact that immune and inflammatory responses are powerfully induced in vivo during therapeutic treatment, thereby substantially limiting the number of administrations which can be envisaged (McCoy, Human Gene Therapy 6 (1995), 1553-1560; Yang, Immunity 1 (1996), 433-442). These many drawbacks, in particular within the context of using viral vectors in man, have led several groups to develop alternative systems for transferring polynucleotides.

Non-viral vectors, which are based on receptor-mediated mechanisms (Wagner Advanced Drug Delivery Review, 14 (1994), 113-135) or on cationic molecules (such as cationic lipids or cationic polymers)-mediated transfection (for a review see Rolland, Critical Review in Therapeutic Drug Carrier Systems 15 (1998), 143-198) promise to have advantages with respect to large scale production, reduced risks related to viral vectors, targeting of transfectable cells, lower immunogenicity and the capacity to deliver larger fragments of nucleic acid.

The development of non-viral vectors for the delivery of nucleic acids into cells necessitates molecules that can associate with nucleic acids to allow these large, hydrophilic polyanions to cross the cell membrane, which is a hydrophobic, negatively charged barrier of a phospholipid bilayer, help them to escape from lysosomal degradation and facilitate their entry into the nucleus, and therefore facilitate the expression of a gene of interest enclosed in said nucleic acid.

Although described as early as 1965 (Bangham, J. Mol. Biol. 13 (1965), 238-252), liposomes, which can encapsulate molecules of interest for delivery to the cell, did not reach the marketplace as injectable therapeutics in humans until the 1990s (Gregoriadis, Trends in BioTechnology 13 (1995), 527-537). This delay is attributable to difficulties in obtaining reproducible formulations with acceptable stabilities. A major breakthrough was the development of "sterically stabilized liposomes" which contained a certain percentage of polyethyleneglycol (PEG)-modified phospholipids (PEG-PLs) (Gregoriadis, 1995). These PEG-PL containing liposomes proved to be more successful in evading detection and elimination by the reticulo-endothelial system, which resulted in greatly enhanced circulation half-lives. While (PEG)-modified liposomes have been most widely used, other hydrophilic polymers such as poly(vinyl pyrrolidone) which augment the stability of liposomes when grafted on their surface have been described (Torchilin, Biochem. Biophys. Acta 1195 (1994), 181-184).

Progress in lipid-mediated nucleic acid transfer into cells was advanced with the introduction of cationic lipids as vehicles for the transfer of nucleic acids into cells (Feigner, Proc. Natl. Acad. Sci. 84 (1987), 7413-7417; Scherman, Current Opinion in Biotechnology 9 (1998), 480-485). Because cationic lipids are positively charged, they are able to complex with negatively charged molecules, and specifically with nucleic acids forming complexes named lipoplexes. Unlike liposomes, these complexes do not require an encapsulation step and are prepared by simple mixing of components. The complexes or lipoplexes essentially comprise a lipid-coated nucleic acid, in which the positively charged coat of the lipoplexes neutralizes the negative charges of the nucleic acid and, also, can efficiently bind to the negatively charged cell surface, facilitating entry of nucleic acid into cell.

Similarly, cationic polymers have been identified which are able to form complexes with negatively charged nucleic acids forming polyplexes and to mediate transfection of nucleic acids into cells. Examples of these cationic polymers are polyamidoamine (Haensler, Bioconjugate Chem. 4 (1993), 372-379), dendritic polymer (WO 95/24221), polyethylene imine or polypropylene imine (WO 96/02655), polylysine (US-A-5,595,897 or FR 2 719 316) or chitosan (US-A-5,744,166).

The advantages of using cationic molecules to mediate transfection of nucleic acids include the simplicity of preparation of the complexes ("lipoplexes", "polyplexes" or association of nucleic acid with cationic lipid and polymer named "lipopolyplexes", hereinafter the three complexes are commonly named "posiplexes"), the ability of the lipid and/or polymer component to complex most of the nucleic acid, a wide range of cell types amenable to transfection, a high efficiency of transfer, lack of immunogenicity of the complexes and availability of the cationic lipids or polymers through chemical synthesis.

Cationic lipid-mediated delivery of nucleic acid to a wide variety of cell types has been demonstrated in vitro and in vivo. For example, nucleic acid encoding the CFTR gene complexed with cationic lipids has been delivered to mouse lungs by intratracheal instillation (Yoshimura, Nucleic Acid Res. 20 (1992), 3233-3240) or by aerosol delivery (Stribling, Proc. Natl. Acad. Sci. 89 (1992), 11277-11281). Human clinical studies with lipoplexes delivery of the CFTR gene to CF patients demonstrated expression of the gene in nasal epithelium and no adverse clinical effects (Caplen, Nature Medicine 1 (1995), 39-46).

Systemic gene expression of a reporter gene following a single intravenous injection of a cationic lipid - DNA complex has been shown in mice (Zhu, Science 261 (1993), 209-211). Moreover, safety studies in rodents and non-human primates of systemically administered cationic lipid-nucleic acid complexes have shown no significant toxicity associated with administering such complexes (Parker, Human Gene Therapy 6 (1995), 575-590).

Cationic lipid-mediated transfection of mRNA in tissue culture was demonstrated to lead to translation of the transcript (Malone, Proc. Natl. Acad. Sci. 86 (1989), 6077-6081). Delivery of antisense oligonucleotides to human endothelial cells using cationic lipids provided increased cellular uptake of the oligonucleotides and increased activity thereof in the cells (Rennet, Mol. Pharm. 41 (1992), 1023-1033). The use of cationic lipids complexed to retroviral particles has allowed viral infection of cells which lacked the appropriate virus receptor (Innes, J. Virol. 64 (1990), 957-962) or enhanced retroviral transduction using complexes known as virosomes (Hodgson, Nature Biotechnol. 14 (1996), 339-342).

Immunotherapy for cancer using lipoplexes containing major histocompatibility (MHC) genes directly injected into mice tumours elicited immune responses that resulted in tumour regression (Plautz, Proc. Natl. Acad. Sci. 90 (1993), 4645-4649). Human clinical studies have been performed using cationic lipid-mediated delivery of DNA sequences encoding immunotherapeutic molecules in man melanoma, colorectal and renal cancer patients (Nabel, Proc. Natl. Acad. Sci. 90 (1993), 11307-11311; Crystal, Science 270 (1995), 404-410).

Similarly, cationic polymer-mediated transfection of plasmid DNA was shown in vivo and in vitro using polyamino polymer (Goldman, Nature Biotech. 15 (1997), 462-466). Substituted polylysines have been employed in order to transfer genes into airway epithelial cells in cystic fibrosis (Kollen, Human Gene Therapy 7 (1996), 1577-1586). Alila, Human Gene Therapy 8 (1997), 1785-1795, also have developed a nonviral gene therapy method to express IGF-I within target muscles by intramuscular injection of plasmid DNA complexed with polyvinylpyrrolidone.

The cationic lipid formulations to date have often incorporated the phospholipid dioleoylphosphatidylethanolamine (DOPE). This phospholipid is thought to disrupt the endosomal membrane by destabilizing its bilayer structure, allowing the lipid-nucleic acid complex to escape endosomal degradation and enter into the cytoplasm (Farhood, Biochem. Biophys. Acta 1235 (1995), 289-295).

However, a significant obstacle in the widespread use of posiplexes for nucleic acid delivery to cells is the tendency of said complexes to be unstable and to form large aggregates in solution, more particularly in the case where charge ratio (+/-) in the posiplex is below 10. Said aggregates have a particule size which is not compatible with their use in gene therapy. Furthermore, studies evaluating the pharmacokinetic behavior of liposomes following intraveinous administration demonstrated that liposome size was a critical determinant of circulation longevity (Senior, Biochim. Biophys. Acta 839 (1985), 1-8; Liu and Huang, Journal of Liposome Research 2 (1992), 57-66). Thus, the technical problem underlying the present invention is to provide means and methods for obtaining stable posiplexes and preparing homogenous suspensions of stable posiplexes.

WO 96/34109 indicates that the order in which individual plasmid DNA and cationic lipid components are mixed to produce plasmid DNA/cationic lipid complexes (lipoplexes) is important to the final results and proposes to prevent lipid aggregation by slow addition of a plasmid DNA solution to a cationic lipid solution at an ionic strength that is lower than isotonicity followed by an adjustment step of lipoplexes to near isotonicity.

WO 96/40962 prevents aggregation of lipoplexes by controlling the ratio of DNA to lipid carrier, minimimizing the overall concentration of DNA: lipid carrier in solution (usually less than 5 mg DNA/ml solution) and avoiding the use of chelating agents such as EDTA and/or significant amounts of salt.

WO 98/08489 proposes to prevent aggregation of lipoplexes by incorporating in the complex particles polyethylene glycol, perfluorinated or partially fluorinated alkyl chains or polyglucuronic acid coupled to a moiety capable of being included into the lipoplexes.

WO 98/34648 prevents the aggregation of posiplexes by including in posiplexes composition at least a non-ionic surfactant having at least one hydrophobic and at least one hydrophilic part, such as for example polyoxyalkylene or a derivative thereof.

The applicant has now identified a new solution to this technical problem which is achieved by the embodiments characterized in the claims, namely the applicant has now developed a method for preparing an homogenous suspension of stable posiplexes, which suspension can be envisaged for use, in particular, in vivo within the context of gene therapy, in particular on account of the harmlessness of its components.

Thus, the present invention relates to stable posiplexes that can be used to deliver nucleic acid to a cell for the purpose of providing a therapeutic molecule to the cells of an individual in need of such treatment. The invention is also directed to stable posiplexes which contain stabilizing additives. The invention is further directed to methods for the preparation of homogenous suspensions of stable posiplexes by combining at least one cationic transfecting molecule, at least one nucleic acids, at least one stabilizing additive and eventually at least one colipid and/or other compound such as peptides or targeting compounds. The invention also includes a method for preparing of homogeneous suspensions of stables posiplexes using optionnally sizing procedures such as extrusion, which can also be used as the final sterilizing step in the production process of posiplexes for administration to patients for therapeutic purposes. The invention is further directed to an homogeneous suspension of stable posiplexes produced by the above methods.

In a first aspect, the present invention relates to a method for preparing an homogeneous suspension of stable posiplexes, comprising:
- combining at least one cationic transfecting molecule, at least one nucleic acid component, at least one stabilizing additive and optionally at least one colipid to form posiplexes,
wherein said stabilizing additive is a polar compound selected from the group consisting of:
a) aprotic polar compounds as defined by: 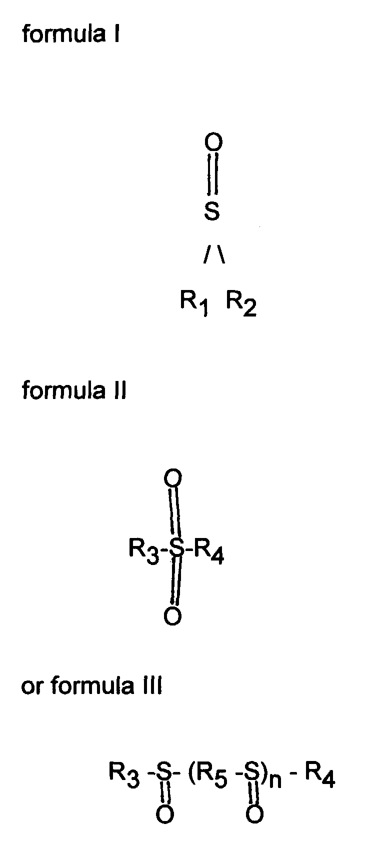 in which R1, R2, R3 and R4 are identical or different and are aryl, alkyl, cycloalkyl, fluoroalkyl, alkenyl or oxyalkyl radicals of 1 to 8 carbon atoms which are optionally repeated, which are linear or branched and which are optionally substituted,
   R5 is (CH₂)x, independently of one another in each [R5-S] n repeat, where x = 1 to 6, with R5 being optionally substituted,
   n = 1 to 50
      with it being possible for R1 and R2 or R3 and R4, respectively, to be linked in order to cyclize the molecule,
b) aprotic polar compounds selected from the group consisting of dimethylformamide, dimethylacetamide, tetramethylurea and their derivatives,
c) protic polar compounds selected from the group defined by: 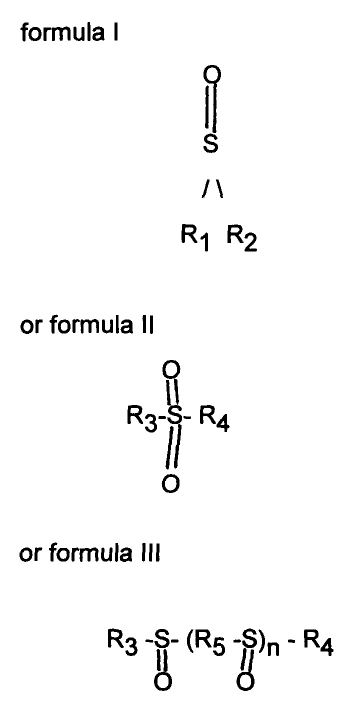 in which R1, R2, R3 and R4 are identical or different and are aryl, alkyl, cycloalkyl, fluoroalkyl, alkenyl, or oxyalkyl radicals of 1 to 8 carbon atoms which are optionally repeated, which are linear or branched and which are substituted by at least one protic group such as, for example, a hydroxyl group,
   R5 is (CH₂)x, independently of one another in each [R5-S] n repeat, where x = 1 to 6, with R5 being optionally substituted,
   n = 1 to 50
      with it being possible for R1 and R2 or R3 and R4 to be linked in order to cyclize the molecule.

Preferably, said radicals of R1, R2, R3 and/or R4 comprise 2 to 6 carbon atoms, more preferably 2 to 4 carbon atoms, and particularly preferred 2 or 3 carbon atoms. Molecules in all possible racemic, enantiomeric and diastereoisomer forms of said formulas are also included in the scope of the present invention.

The expression "alkenyl" is understood as meaning that the carbon chain can include one or more double bond(s) along the said chain.

According to a preferred embodiment, said stabilizing additive is an aprotic polar compound and is selected from the group consisting of di-ethyl sulfoxide (DESO), di-methyl sulfoxide (DMSO), di-n-propyl sulfoxide (DPSO), tetramethylene sulfoxide (TEMSO), dimethylsulphone, sulpholane, 1-methyl-2-pyrrolidone and their derivatives.

In accordance with the present invention, "aprotic polar compound" is understood as referring to a compound which does not contain any positively polarised hydrogen atoms (aprotic). The properties of such compounds are widely described in the literature (see, for example, Vollhardt and Schore, 1994, Traité de Chimie Organique [Treatise on Organic Chemistry], 2nd edition, Ed. De Boeck University). Such compounds can, in particular, be obtained naturally or synthetically, or by the chemical modification of a first compound which does not initially exhibit the abovementioned properties. The skilled person is in possession of the necessary knowledge to identify such compounds. The aprotic polar compounds to be employed in the compositions, methods and uses of the present invention such as those described below may be obtained from various commercial sources or produced as described in the prior art. A large number of polar compounds can be considered for use within the context of the invention. According to a preferred embodiment, the aprotic polar compound is in aqueous solution, that is to say it is diluted in an aqueous solution which is, where appropriate, saline and buffered. The skilled person is in possession of sufficient knowledge to enable him to select the aqueous solution which is most appropriate for the targeted cell type. More specifically, the said polar compound is in solution in water or in a buffer, for example 20 mM Hepes, pH 7.5.

According to the invention, the R1, R2, R3, R4 and/or R5 groups of the polar compounds can be substituted. Such substitutions can, in particular, consist of a labelling molecule (see labelling molecules in US-A-4,711,955) enabling, for example, visualization of the distribution of the compounds, or of complexes incorporating them, after in vitro or in vivo administration; a cell targeting molecule (ligand) or an anchoring molecule. These elements, which have been widely described in scientific publications, allow targeting of a specific cell type, facilitating penetration into the cell, lysis of endosomes or even intracellular transport towards the nucleus. These elements may be composed of all or part of sugars, glycol, peptides (e.g. GRP, Gastrin Releasing Peptide), oligonucleotides, lipids, hormones, vitamins, antigens, antibodies (or fragments thereof), specific membrane receptor ligands, ligands capable of reaction with an anti-ligand, fusogen peptides, nuclear localization peptides, moiety capable of being incorporated into the posiplex such as phospholipid, or a combination of said compounds, e.g. galactosyl residues to target the asialoglycoprotein receptor on the surface of hepatocytes, the INF-7 fusogen peptide derivated from the HA-2 subunit of the influenza virus hemagglutinin (Plank, J. Biol. Chem. 269 (1994), 12918-12924) for membrane fusion, or a nuclear signal sequence derived from the T-antigen of the SV40 virus (Lanford, Cell 37 (1984), 801-813) or from the EBNA-1 protein of the Epstein Barr virus (Ambinder, J. Virol. 65 (1991), 1466-1478).

For the purpose of the present invention "derivative" of any of the aforementioned compounds means molecules the chemical structure of which is based on that of the above described compounds and which can be used for stabilizing posiplexes according to the present invention. Methods for the preparation of such derivatives are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, computer aided design of appropriate derivatives and analogues can be used, for example, according to methods known in the art. Furthermore, a three-dimensional and/or crystallographic structure of DMSO and other related aprotic polar compounds can be used for the design of compounds capable of stabilizing additive (Rose, Biochemistry 35 (1996), 12933-12944; Rutenber, Bioorg. Med. Chem. 4 (1996), 1545-1558).

According to the invention, "cationic transfecting molecule" means a cationic lipid or/and a cationic polymer. It should be understood that said cationic lipid and polymer can be independant of one another, or linked by chemical modification. In a particular embodiment of the invention, the cationic transfecting molecule used in the claimed method can be at least one cationic lipid, at least one cationic polymer or a mixture of at least one cationic lipid and of at least one cationic polymer. Because cationic transfecting molecules are positively charged, they are able to complex with negatively charged nucleic acids forming complexes or particles.

According to the present invention, "posiplex" is a generic name for referring to "lipoplex", "polyplex or/and lipopolyplex". "lipoplex" means a cationic lipid-based nucleic acid complex or particle. Such complexes or particles usually contain at least one cationic lipid and at least one nucleic acid component. "polyplex" means a cationic polymer-based nucleic acid complex or particle. Such complexes or particles usually contain at least one cationic polymer and at least one nucleic acid component. "lipopolyplex" means a cationic lipid/polymer mixture-based nucleic acid complex or particle. Such complexes or particles usually contain at least one cationic lipid, at least one cationic polymer and at least one nucleic acid component.

Said posiplexes can further comprise at least one stabilizing additive and optionally at least one colipid, and /or other compounds such as for example peptides, sugar, targeting compound, PEG, lipid-based compound (ex. DSPE-PEG).

"Stable posiplexes" means that independently of their size said posiplexes do not form aggregates and/or precipitate in solution, especially in aqueous solution.

"Homogeneous suspension" is notably the result of a suspension containing non-aggregated and/or non-precipitated complexes or particles, e.g. posiplexes. The term "homogeneous suspension" in the scope of the present invention means, in this regard, that said suspension becomes less or not flocculent or precipitated when the stabilizer additive is present compared to a suspension prepared without addition of a stabilizer additive. This can be determined by comparing the amount of aggregation or precipitation observed without the use of a stabilizer additive with the amount observed in the presence of said stabilizer additive under the same experimental conditions. Homogeneity of a suspension can be determined upon visual inspection of the obtained suspension which become rapidly flocculent or precipitated if the stabilizer additive is omitted, or can be achieved by a number of techniques including, but not limited to, dynamic laser light scattering (photon correlation spectrocopy, PCS), electronic microscopy, freeze fracture electronic microscopy as well as other techniques known to those skilled in the art (see, Washington, Particle Size Analysis in Pharmaceutics and other Industries, Ellis Horwood, New York, 1992, 135-169). "Homogeneous suspension of stable posiplexes" may refer to suspension of stable "lipoplexes", stable "polyplexes", stable "lipopolyplexes" or a mixture of at least two of these ones.

According to a particular embodiment, said invention relates to a method wherein said method further comprises combining said cationic transfecting molecule, said nucleic acid, said stabilizing additive, and optionally said co-lipid, with at least one compound selected in the group consisting of targeting compounds and labelling compounds.

"Targeting compounds" and "labelling compounds" means a natural or synthetic component or molecule which is able to allow targeting of a specific cell type, facilitating penetration into the cell, lysis of endosomes or even intracellular transport towards the nucleus and to enable visualization of the distribution of the complexes incorporating them, respectively. Such compounds are widely disclosed in litterature, and some examples have been provided above.

According to the present invention, homogeneous suspension of stable posiplexes may advantageously be homogeneously sized by an optional step consisting in a sizing procedure. Therefore, the present invention further relates to a method as previously described which further comprises an optional step consisting in a sizing procedure. Methods which can be used in this sizing step include, but are not limited to, extrusion, sonication and microfluidization, size exclusion chromatography, field flow fractionation, electrophoresis and ultracentrifugation.

In a preferred embodiment, said sizing step comprises extruding the cationic transfecting molecule or of the posiplex through membranes of defined pore diameter. An extruder may be used in which polycarbonate membranes of defined pore diameter are stacked so that the suspension is forced through the membranes under pressure (for example, from Lipex Biomembranes, Inc., Vancouver, Canada). Cationic transfecting molecule or posiplex may be extruded through membranes having pore sizes in the range of 50 to 500 nm. Preferred membranes have a pore diameter of 200-300 nm. Extrusion of the posiplexes can also be used as the final sterilizing step in the production process of posiplexes for administration to patients for therapeutic purposes.

The invention is also directed to methods for preparing an homogeneous suspension of stable posiplexes as previously described which further comprise a sizing step of the cationic transfecting molecule before addition of nucleic acids using sizing methods standardizing particle size of said cationic transfecting molecule such as those above disclosed.

According to a particular embodiment, the method of the present invention is characterized in that said cationic transfecting molecule, and optionally said colipid, are combined with said nucleic acid prior combination with said stabilizing additive.

According to another particular embodiment, the method of the present invention is characterized in that said cationic transfecting molecule, or said nucleic acid, is combined with said stabilizing additive before combining it with said nucleic acid, or said cationic transfecting molecule, respectively.

In a preferred embodiment of the invention, the posiplexes in the homogeneous suspension has a complex or particle size of 500 nm or less, preferably 300nm or less. Particle size may be selected for optimal use in a particular application.

Measurements of the posiplexes size can be made by a number of techniques including, but not limited to, dynamic laser light scattering (photon correlation spectroscopy, PCS), as well as other techniques known to those skilled in the art; see for example Washington, Particle Size Analysis in Pharmaceutics and other Industries, Ellis Horwood, New York, 1992, pp 135-169.

After the posiplexes have been subjected to a sizing procedure, e.g., extrusion, the yield percentage may be calculated to assess the recovery. This calculation is based on determining the concentration of nucleic acid in the posiplexes before and after the procedure. For example, where a suspension of posiplexes is extruded through sizing membranes, the DNA concentration in the suspension may be determined using standard techniques, e.g., measurement of absorbance at 260 nm, which detects nucleic acids.

Another beneficial effect of the stabilizing additives of the invention could be to maintain the integrity of the posiplexes, to maintain posiplexes stability during the sizing procedures if any, and to increase shelf life.

According to the method of the present invention, the cationic transfecting molecule is a cationic lipid or a cationic polymer.

Cationic lipid or mixture of cationic lipids which may be used in the method of the present invention includes cationic lipid selected from the group consisting of Lipofectin™, DOTMA: N-[1-(2,3-dioleyloxyl)propyl]-N,N,N-trimethylammonium (Felgner, PNAS 84 (1987), 7413-7417), DOGS: dioctadecylamidoglycylspermine or Transfectam™ (Behr, PNAS 86 (1989), 6982-6986), DMRIE: 1 2-dimiristyloxypropyl-3-dimethyl-hydroxyethylammonium and DORIE: 1,2-diooleyloxypropyl-3-dimethyl-hydroxyethylammnoium(Felgner, Methods 5 (1993), 67-75), DC-CHOL: 3 [N-(N',N'-dimethylaminoethane)-carbamoyl]cholesterol (Gao, BBRC 179 (1991), 280-285), DOTAP (McLachlan, Gene Therapy 2 (1995), 674-622), Lipofectamine™, spermine or spermidine-cholesterol, Lipofectace™ (for a review see for example Legendre, Medecine/Science 12 (1996), 1334-1341 or Gao, Gene Therapy 2 (1995), 710-722), cationic lipid as disclosed in patent applications WO 98/34910, WO 98/14439, WO 97/19675, WO 97/37966, FR 97 02420 (filing number)or FR 97 07290 (filing number) and their isomers. Nevertheless, this list is not exhaustive and other cationic lipids well known in the art could be used in the method of the invention as well.

Preferably, the cationic lipids of the present invention are selected from among cationic lipids of formula (WO 98/34910): 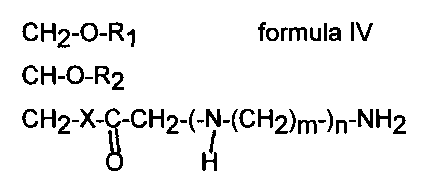 wherein:
R₁, R₂, are identical or different and are C₆-C₂₃ alkyl or alkenyl, linear or branched, or -C(=O)-(C₆-C₂₃)alkyl or -C(=O)-(C₆-C₂₃)alkenyl, linear or branched,
X is O or -NR₃, R₃ being H or C₁-C₄ alkyl,
n = 1 to 6,
m = 1 to 6, and when n > 1, m can be identical or different.

According to another embodiment, the cationic lipid is selected from among cationic lipids of formula (FR 97 02420 filing number):

R-HN-[-(CH₂)ₘ - NR-]_{n₋1}-(CH₂)ₘ-NH-R formula V

wherein:
R is, independently of one another, H or 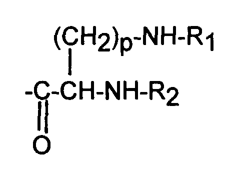 wherein:
R₁ and R₂ are, independently of one another C₆-C₂₃ alkyl or alkenyl, linear or branched, or -C(=O)-(C₆-C₂₃) alkyl or -C(=O)-(C₆-C₂₃)alkenyl, linear or branched, aryl, cycloalkyl, fluoroalkyl, polyethylene glycol, oxyethylene ou oxymethylene radicals,
p = 1 to 4,
n = 1 to 6,
m =1 to 6.

Cationic polymers or mixture of cationic polymers which may be used in the method of the present invention includes cationic polymer selected from the group consisting of chitosan, poly(aminoacids) such as polylysine (US-A-5,595,897 and FR 2 719 316); polyquaternary compounds; protamine; polyimines;polyethylene imine or polypropylene imine (WO 96/02655) ; polyvinylamines; polycationic polymer derivatized with DEAE, such as complexes of DEAE with methacrylate, dextran, acrylamide, polyimines, albumin, pullulans, celluloses; polyvinylpyridine; polymethacrylates; polyacrylates; polyoxethanes; polythiodiethylaminomethylethylene (P(TDAE)); polyhistidine; polyornithine; poly-p-aminostyrene; polyoxethanes; co-polymethacrylates (eg copolymer of HPMA; N-(2-hydroxypropyl)-methacrylamide); compound disclosed in US-A-3,910,862, polyvinylpyrrolidonedimethylaminomethylmethacrylates and polyvinylpyrrolidonemethylacrylaminopropyltrimethyl ammonium chlorides; polyamidoamines; telomeric compounds (patent application filing number EP 98.401471.2). Nevertheless, this list is not exhaustive and other cationic polymers well known in the art can be used in the method of the invention as well.

According to a particular embodiment, the cationic polymer is a substituted polyvinylamine (PCT patent application, filing number PCT/FR98/01581) such as defined by formula: 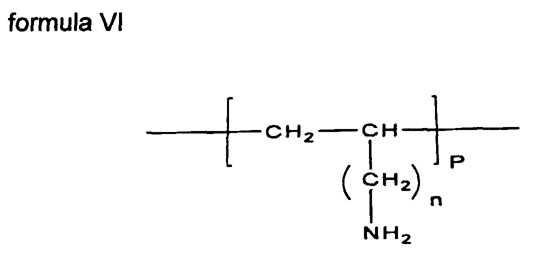 wherein n =0 to 5, p = 2 to 20000
wherein:
- at least 10%, of free NH₂ are substituted with R, and R is an hydrophilic group.

According to another embodiment said cationic polymer is a polymer of the general formula (european patent application, filing number 98/402142.8): 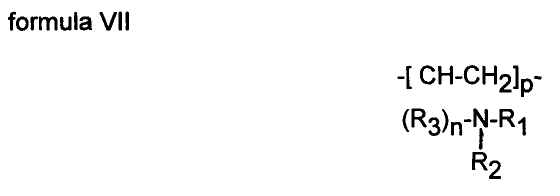 wherein:
the degree of polymerization p ranges from 2 to 1000000;
R₁, R₂ and R₃, independently of one another in each [CH - CH₂] repeat, are selected from H, alkyl of 1 to 20 carbon atoms or aryl of 5 to 7 carbon atoms; n is 0 or 1, with the proviso that at least one n is 1 in the full length of the polymer.

Colipids are optionally included in the posiplexes in order to facilitate entry of the nucleic acid into the cell or to conjugate stabilizing additive according to the invention. According to methods of the invention, colipids are selected in the group consisting in positively or negatively charged, neutral and zwitterionic lipids. These colipids could be natural or synthetic compounds, or a combination of each.

Preferrably, the colipid is selected from the group consisting in positively of phosphatidylethanolamine (PE), phosphatidyicholine, phosphocholine, dioleylphosphatidylethanolamine (DOPE), sphingomyelin, ceramide or cerebroside or one of their derivates.

The ratios of cationic transfecting molecule to colipid (on a weight to weight basis) can range from 1:0 to 1:10. In preferred embodiments, the ratio ranges from 1:0.5 to 1:4.

According to a specific embodiment, the stabilizing additive of the methods of the invention is coupled to a moiety capable of being incorporated into the complex or particle. Colipid may be added to the posiplexes of the invention to allow the attachment of stabilizing additives to said posiplexes. The colipid can be a moiety that allows the stabilizing additive to be incorporated into the posiplex. Derivatization of the lipid with an additive allows the moiety to anchor the stabilizing additive to the posiplexes. The colipid can be conjugated to the additive which prevents aggregation, floculation and/or precipitation of posiplexes.

Colipids, or moieties, which may be used to incorporate such additives to the posiplexes of the invention include, but are not limited to, zwitterionic or other phospholipids. Preferably, the colipid used to conjugate a stabilizing additive is a moiety capable of being incorporated into the posiplexes. More preferably, such a colipid is inert and biocompatible.

According to a particular embodiment, the cationic transfecting molecule used in the method of the invention may be synthesized to contain stabilizing additives such as those previously described.

"Polynucleotide or nucleic acid" is understood as meaning a naturally isolated or synthetic, linear or circular, double-stranded or single-stranded DNA and/or RNA fragment, with the term designating a precise sequence of labelled or unlabelled (see, for example, US-A-4,711,955 or EP-A-0 302 175), modified or unmodified (see, by way of example, US-A-5,525,711) nucleotides and making it possible to define a fragment or a region of a nucleic acid without limiting its size. Nucleic acid is understood as referring, in particular, to a cDNA; to a genomic DNA; to a plasmid DNA; to a nucleic acid which is free of any compound which facilitates its introduction into cells; to a nucleic acid which is associated with at least one polypeptide, in particular a polypeptide of viral origin, more particularly of adenoviral or retroviral origin, or with a synthetic polypeptide; to a nucleic acid which is associated with a ligand; to a nucleic acid which is associated with at least one cationic amphiphile, in particular lipids; to a nucleic acid which is associated with at least one cationic or neutral polymer; to a messenger RNA; to an antisense RNA; to a ribozyme; to a transfer RNA; to a ribosomal RNA; or to a DNA which encodes such RNAs.

According to one preferred embodiment of the invention, the said nucleic acid comprises at least one gene of interest and elements which enable the said gene of interest to be expressed. In this embodiment, the said nucleic acid is advantageously in plasmid form. The elements which enable expression to take place are the totality of the elements which enable the said DNA fragment to be transcribed into RNA (antisense RNA or mRNA) and the mRNA to be translated into polypeptide. These elements are, in particular, promoter sequences and/or regulatory sequences which are effective in the said cell and, where appropriate, the sequences which are required for enabling the said polypeptide to be secreted or expressed at the surface of the target cells. By way of example, mention may be made of the promoters of the RSV, MPSV, SV40, CMV or 7.5 k viruses, or of vaccinia virus, or the promoters of the genes which encode muscle creatine kinase, actin and pulmonary surfactant. It is furthermore possible to select a promoter sequence which is specific for a given cell type or which can be activated under defined conditions. The literature provides a great deal of information relating to such promoter sequences. Furthermore, the said nucleic acid can include at least two identical or different sequences exhibiting transcriptional promoter activity and/or at least two identical or different DNA coding sequences which are located contiguously or at a distance, and in the same direction or in the opposite direction, with respect to each other without the function of the transcriptional promoter or the transcription of the said sequences being affected thereby. Similarly, it is possible, in this type of nucleic acid construct, to introduce "neutral" nucleic acid sequences or introns which do not affect transcription and which are spliced before the translation stage. Sequences of this nature, and their uses, are described in the literature. The said nucleic acid can also encompass sequences which are required for intracellular transport, for replication and/or for integration. Such sequences are well known to the skilled person. In addition, the nucleic acids according to the present invention can also be nucleic acids which are modified such that they are unable to integrate into the genome of the target cell, or nucleic acids which are stabilized with agents such as, for example, spermine.

The suspension/composition of the invention may further comprise a selectable marker gene, either linked to the above described nucleic acids or as separate nucleic acid molecules, e.g. in a recombinant plasmid. This embodiment is particularly suited for ex vivo treatment of tissue, cells and organs. Following the introduction of foreign DNA, engineered cells may be allowed to grow for 1-2 days in an enriched media, and then are switched to a selective media. The selectable marker in the recombinant plasmid confers resistance to the selection and allows for the selection of cells having stably integrated the plasmid into their chromosomes and grow to form foci which in turn can be cloned and expanded into cell lines. A number of selection systems may be used, including but not limited to the herpes simplex virus thymidine kinase (Wigler, Cell 11 (1977), 223), hypoxanthine-guanine phosphoribosyltransferase (Szybalska, Proc. Natl. Acad. Sci. USA 48 (1962), 2026), and adenine phosphoribosyltransferase (Lowy, Cell 22 (1980), 817) in tk⁻, hgprt⁻ or aprt⁻ cells, respectively. Also, antimetabolite resistance can be used as the basis of selection for dhfr, which confers resistance to methotrexate (Wigler, Proc. Natl. Acad. Sci. USA 77 (1980), 3567; O'Hare, Proc. Natl. Acad. Sci. USA 78 (1981), 1527), gpt, which confers resistance to mycophenolic acid (Mulligan, Proc. Natl. Acad. Sci. USA 78 (1981), 2072); neo, which confers resistance to the aminoglycoside G-418 (Colberre-Garapin, J. Mol. Biol. 150 (1981), 1); hygro, which confers resistance to hygromycin (Santerre, Gene 30 (1984), 147); or puromycin (pat, puromycin N-acetyl transferase). Additional selectable genes have been described, for example, trpB, which allows cells to utilize indole in place of tryptophan; hisD, which allows cells to utilize histinol in place of histidine (Hartman, Proc. Natl. Acad. Sci. USA 85 (1988), 8047); and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.).

Suitable vectors and plasmids useful to be employed in the suspension/composition of the invention for in vitro or in vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. Preferably, said vector is a gene transfer or targeting vector. Methods which are well known to those skilled in the art can be used to construct the above mentioned nucleic acids, plasmids and recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989), (1994).

Within the context of the present invention, the nucleic acid can be homologous or heterologous to the target cell. It can be advantageous to use a nucleic acid which encodes all or part of a polypeptide, in particular a polypeptide which exhibits a therapeutic or prophylactic activity, more specifically an immunogenic activity of the cellular or humoral type. The term polypeptide is to be understood as being without restriction with regard to the size of the polypeptide or the degree to which it is modified (for example by glycosylation). By way of example, mention may be made of the genes which encode an enzyme, a hormone, a cytokine, a membrane receptor, a structural polypeptide, a polypeptide which forms a membrane channel, a transport polypeptide, an adhesion molecule, a ligand, a factor for regulating transcription, translation, replication or transcript stabilization, or an antibody, such as, for example, the gene which encodes the CFTR protein, dystrophin, factor VIII or factor IX, HPV E6/E7, MUC1, BRAC1, interferon, the interleukins (IL-2, IL-4, IL-6, IL-7 and IL-12), tumour necrosis factor (TNF) alpha or GM-CSF (granulocyte macrophage colony stimulating factor), or the tk gene of herpes simplex virus type 1 (HSV-1), the retinoblastoma gene or the gene for p53, or all or part of immunoglobulins, such as the F(ab)2, Fab' and Fab fragments, or the anti-idiotype immunoglobulins (US-A-4,699,880). It will, of course, be understood that this list is not limiting and that other genes can be employed.

According to the present invention, it may be desirable to obtain a stable posiplex which comprises the highest possible concentration of nucleic acid in order to be able, if necessary, to administer the smallest possible volume of suspension/composition according to the invention. The skilled person is in possession of adequate knowledge to enable him to adjust this concentration in accordance with the solubilizing power of the medium in which the said nucleic acid is dissolved.

In a preferred embodiment using expressible nucleic acid, plasmid DNA is used. Concentrations of nucleic acid whixh may be added to the cationic lipids to form posiplexes of the invention range from 10 µg/ml to 1000 µg/ml. In a preferred embodiment of the invention, the concentration of nucleic acid ranges from 20 µg/ml to 500 µg/ml.

The posiplexes may also be characterized by their charge ratio (+/-), which is the ratio of the positively charged cationic lipid component to the negatively charged nucleic acid component of the posiplex. In general, an excess positive charges of the posiplex facilitates its binding to the negatively charged cell surface. The charge ratio of a complex of the invention may be calculated by dividing the sum of the positive charges by the negative charges of the posiplex. A determination of the positive charges per mole can be made for a specific cationic lipid or colipid such that a given weight of lipid will represent a specific positive charge. An analagous determination can be made with respect to the nucleic acid or colipid to yield the negative charge per mole so that a given weight of nucleic acid or colipid will represent a specific negative charge. By dividing all positive charges by all negative charges, the net charge ratio (+/-) of the posiplex is determined.

Preferably, the range of the charge ratio of the posiplexes of the invention are from 1 to 20 (+/-). More preferably, the range is from 2.5 to 10 (+/-).

The invention is further directed to stable posiplexes and homogeneous suspension thereof produced by the methods previously described. According to a specific embodiment, the volume of stabilizing additive combined with the other components, and therefore its volume in the homogeneous suspension prepared by said method, can represent from 0.05 to 99%, more especially from 1 to 50%, advantageously from 2 to 15% of the total volume of said suspension.

The present invention preferably encompasses posiplexes comprising one or more cationic lipid, one or more stabilizing additive, one or more nucleic acid component, optionally one or more colipid wherein said stabilizing additive is a polar compound selected from the group consisting of:
a) aprotic polar compounds as defined by: 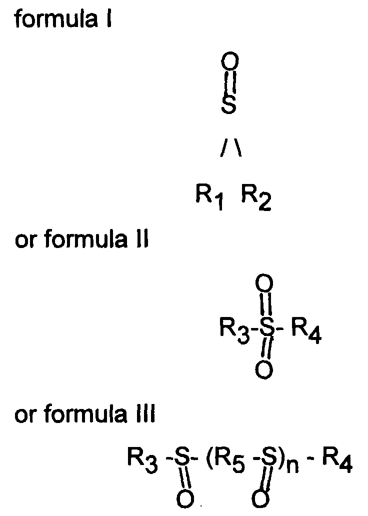 in which R1, R2, R3 and R4 are identical or different and are aryl, alkyl, cycloalkyl, fluoroalkyl, alkenyl or oxyalkyl radicals of 1 to 8 carbon atoms which are optionally repeated, which are linear or branched and which are optionally substituted,
   R5 is (CH₂)x,independently of one another in each [R5-S] n repeat, where x = 1 to 6, with R5 being optionally substituted,
   n = 1 to 50
      with it being possible for R1 and R2 or R3 and R4, respectively, to be linked in order to cyclize the molecule,
b) aprotic polar compounds selected from the group consisting of dimethylformamide, dimethylacetamide, tetramethylurea and their derivatives,
c) protic polar compounds selected from the group defined by: 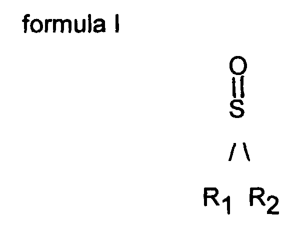 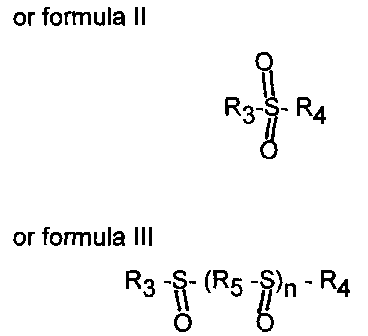 in which R1, R2, R3 and R4 are identical or different and are aryl, alkyl, cycloalkyl, fluoroalkyl, alkenyl, or oxyalkyl radicals of 1 to 8 carbon atoms which are optionally repeated, which are linear or branched and which are substituted by at least one protic group such as, for example, a hydroxyl group,
   R5 is (CH₂)x,independentIy of one another in each [R5-S] n repeat, where x = 1 to 6, with R5 being optionally substituted,
   n = 1 to 50
      with it being possible for R1 and R2 or R3 and R4 to be linked in order to cyclize the molecule.

In a preferred embodiment, the stabilizing additive present in the posiplexes of the invention is an aprotic polar compound and is selected from the group consisting of di-ethyl sulfoxide (DESO), di-methyl sulfoxide (DMSO), di-n-propyl sulfoxide (DPSO), tetramethyl sulfoxide (TEMSO), dimethylsulphone, sulpholane, 1-methyl-2-pyrrolidone and their derivatives.

According to a particularly preferred embodiment, the posiplexes of the invention shows a complex or particle size of 500 nm or less, preferably of 300 nm or less. The invention also relates to a composition comprising at least one aformentioned homogeneous suspension of stable posiplexes or at least one posiplex as previously described and a pharmaceutically acceptable carrier.

Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods.

The present invention is also directed to the use of a said homogeneous suspension of stable posiplexes or at least one of said posiplexes for preparing a composition for therapeutic, prophylactic or vaccination purposes, which composition is intended for treating the human or animal body by means of gene therapy. According to a special embodiment, said composition is intended to be administered in a form which can be injected by the intramuscular route or to be administered by inhalation, by intratracheal injection, by instillation or by aerosolization of the said composition.

According to the invention, these compositions can be administered to the subject at a suitable dose. Administration of the suitable compositions may be effected by different ways, e.g., by intravenous, intratracheal, intrapulmonary, intraperitoneal, subcutaneous, intramuscular, topical or intradermal administration. The dosage regimen will be determined by the attending physician and other clinical factors. As is well known in the medical arts, dosages for any one patient depends upon many factors, including the patients size, body surface area, age, the particular substance to be administered, sex, time and route of administration, general health, and other drugs being administered concurrently. Generally, the regimen as a regular administration of the composition should be in the range of 1 µg to 10 mg as the bioactive compound per day. If the regimen is a continuous infusion, it should also be in the range of 1 µg to 10 mg as the bioactive compound per kilogram of body weight per minute, respectively. Progress can be monitored by periodic assessment. Dosages will vary but a preferred dosage for intravenous administration of nucleic acids, e.g., DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by catheter to a site in an artery. Furthermore, the compositions of the invention can be bound to microcapsules or microspheres before injection. One may either use an in vivo gene-transfer approach for which multiple devices, like double balloon or other catheters have been designed or via direct injection into the targeted tissue as described above. Alternatively it is possible to use an ex vivo approach isolating cells which are known to lodge in tissues from the body which are then transfected using one of the above mentioned compositions and reinjected.

In a still further embodiment, the present invention relates to a vaccine comprising any one of the aforementioned suspensions/compositons. In this embodiment the therapeutically active substance is preferably an antigen or a nucleic acid encoding the same capable of generating a protective immunological response to a disease in a human or an animal susceptible to such disease. The vaccines of the present invention can be prepared according to methods well-known in the art. Immunogenicity of antigens used for vaccination can be enhanced, for example, programming either a Th1- or a Th2-directed immune response, e.g., by co-transducing cDNA coding for secreted cytokines or chemokines or membrane molecules. The vaccines can be, e.g., injected either intradermally, subcutaneously, intramuscularly or, particularly in case of anti-tumor vaccination, into areas of tumor growth or into lymphatic vessels or lymph nodes draining areas of tumor growth.

In another embodiment the present invention relates to a diagnostic composition comprising any one of the above described suspensions/compositions of the invention and optionally suitable means for detection. In this embodiment, the therapeutically active substance is preferably labled. There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds. In addition or alternatively, the therapeutically active substance comprises a marker gene encoding a directly or indirectly detectable protein such as (beta-galactosidase, green fluorescent protein or luciferase). The diagnostic composition of the invention can advantageously be used for targeting, e.g., light emission to selected regions, as well as for tracking entities within the subject. In addition, animal models for disease states can be accomplished by using the above described compositions, as are methods for localizing and tracking the progression of disease or a pathogen within the animal, and for screening putative therapeutic compounds effective to inhibit the disease or pathogen. Methods for detecting and localizing light originating from a mammal are described in the prior art, e.g., WO 97/18841 and references cited therein.

The present invention also relates to the use of a suspension/composition according to the invention for transferring at least one nucleic acid, into target cells in vitro, ex vivo or in vivo, more especially in vivo.

The introduction or transfer process is by itself well known. "transferring or transfer" means that the therapeutically active substance is transferred into the cell and is located, at the end of the process, inside said cell or within or on its membrane. If the active substance is a nucleic acid, this is called "transfection". Transfection can be verified by any appropriate method, for example by measuring the expression of said gene or by measuring the concentration of the expressed protein.

"Target cells" according to the invention are understood as meaning prokaryotic cells, yeast cells and eukaryotic cells, plant cells, human or animal cells and, in particular, mammalian cells. In addition, cancerous cells should also be mentioned. In vivo, the invention can be applied within the interstitial or luminal space of tissues such as the lung, the trachea, the skin, muscle, the brain, the liver, the heart, the spleen, bone marrow, the thymus, the bladder, lymph, blood, blood vessels, the pancreas, the stomach, the kidney, the ovaries, the testicles, the rectum, the peripheral or central nervous system, the eyes, the lymphatic organs, cartilage and endothelium. According to an advantageous choice of the invention, the target cell is a muscle cell, a haematopoietic stem cell or else an airways cell, more especially a tracheal or pulmonary cell, advantageously a respiratory epithelium cell.

The invention also relates to a process for transferring a therapeutically active substance into a target cell, according to which the said cell is brought into contact with a composition according to the invention.

In particular, the compositions of the invention can be used for implementing a method of therapeutic treatment which consists in transferring at least one nucleic acid, into target cells. Preferably, these target cells are mammalian cells. The mammalian cells are, in particular, lung cells, muscle cells or else haematopoietic stem cells.

According to the present invention, and within the context of an in vivo gene therapy, it is possible to repeat the proposed procedure several times, in a given patient, without any significant immune reaction being triggered against one of the administered compounds. The administration can take place in one single dose or in doses which are repeated once or several times at particular intervals. The appropriate route of administration and dosage vary in accordance with a variety of parameters, for example the individual or the disease to be treated, or else the nucleic acid to be transferred.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools. html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The methods, compositions, uses of the invention can be used for the treatment of all kinds of diseases the treatment and/or diagnostic of which being related to or dependent on the transfer of nucleic acids in cells. The compositions, and uses of the present invention may be desirably employed in humans, although animal treatment is also encompassed by the uses described herein.

The invention is illustrated by reference to the followings examples.

### Example 1: Stabilization of lipoplexes by polar non-protic compounds

The lipoplex-stabilizing effect of polar aprotic compounds can be analyzed with different lipoplexes obtained with various lipidic systems:
- System-1:: DOTAP/Chol (1:1) N/P=2.2
- System-2:: pcTG90/DOPE (1:1) N/P=5
- System-3:: DOGS/DOPE (1:1) N/P=5
PcTG 90 is described in patent application WO 98/34910

Lipoplexes consisting of plasmid DNA (pcTG11033 or/and pCMV-luc; in both cases the luciferase gene is under control of promoter sequence from CMV) and of the above cationic lipids and colipids (System-1 to System-3) are formulated at 0.25 mg DNA/ml and the indicated amounts of DMSO (final concentration) are added to the formulated lipoplexes:
System-1, 0.25mg DNA/ml with 0% DMSO, 2%DMSO, 5% DMSO, or 10% DMSO
System-2, 0.25mg DNA/ml with 0% DMSO, 2%DMSO, 5% DMSO, or 10% DMSO
System-3, 0.25mg DNA/ml with 0% DMSO, 2%DMSO, 5% DMSO, or 10% DMSO

To assure reproducibility, samples are prepared in triplicate (3 x 3 x 4 = 36 individual samples) and analysis is carried out by determination of particle size (quasi-elastic laser light scattering at 90° on day-1, day-3, day-7, and day-14, as well as by in vitro transfection assay with detection of luciferase activity on day-1, day-7, and day-14.

## Claims

1. A method for preparing stable posiplexes or an homogeneous suspension thereof, comprising combining at least one cationic transfecting molecule, at least one nucleic acid component, at least one stabilizing additive and optionally at least one colipid to form posiplexes, wherein said stabilizing additive is a polar compound selected from the group consisting of:
a) aprotic polar compounds as defined by: 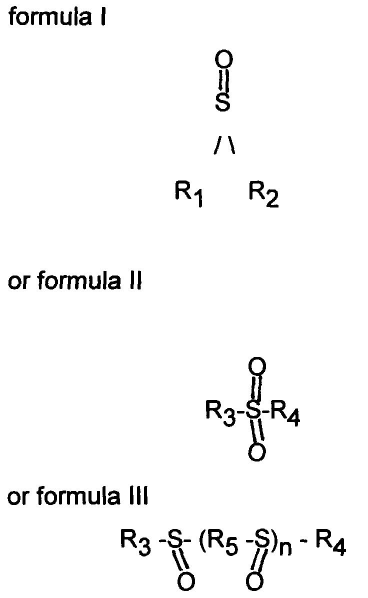 in which R1, R2, R3 and R4 are identical or different and are aryl, alkyl, cycloalkyl, fluoroalkyl, alkenyl or oxyalkyl radicals of 1 to 8 carbon atoms which are optionally repeated, which are linear or branched and which are optionally substituted,
R5 is (CH₂)x,independently of one another in each [R5-S] n repeat, where x = 1 to 6, with R5 being optionally substituted,
n = 1 to 50
with it being possible for R1 and R2 or R3 and R4, respectively, to be linked in order to cyclize the molecule,
b) aprotic polar compounds selected from the group consisting of dimethylformamide, dimethylacetamide, tetramethylurea and their derivatives,
c) protic polar compounds selected from the group defined by: 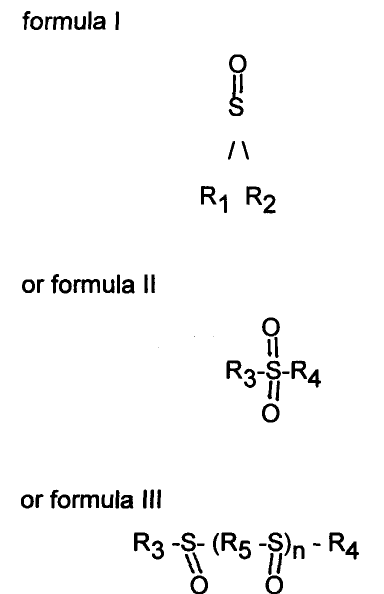 in which R1, R2, R3 and R4 are identical or different and are aryl, alkyl, cycloalkyl, fluoroalkyl, alkenyl, or oxyalkyl radicals of 1 to 8 carbon atoms which are optionally repeated, which are linear or branched and which are substituted by at least one protic group,
R5 is (CH₂)x,independently of one another in each [R5-S] n repeat, where x = 1 to 6, with R5 being optionally substituted,
n = 1 to 50
with it being possible for R1 and R2 or R3 and R4 to be linked in order to cyclize the molecule.

2. The method of claim 1, wherein said stabilizing additive is an aprotic polar compound and is selected from the group consisting of di-ethyl sulfoxide (DESO), di-methyl sulfoxide (DMSO), di-n-propyl sulfoxide (DPSO), tetramethylene sulfoxide (TEMSO), dimethylsulphone, sulpholane, 1-methyl-2-pyrrolidone and their derivatives.

3. The method of claim 1 or 2, wherein said method further comprises combining said cationic transfecting molecule, said nucleic acid, said stabilizing additive, and optionally said co-lipid, with at least one targeting compounds and labelling compounds.

4. The method of any one of claims 1 to 3, wherein said method further comprises an optional step consisting in a sizing procedure.

5. The method of claim 4, wherein the sizing procedure comprises extruding the posiplex through membranes having pore sizes in the range of 50 to 500 nm.

6. The method of any one of claims 1 to 5, wherein said cationic transfecting molecule, and optionally said colipid are combined with said nucleic acid prior combination with said stabilizing additive.

7. The method of any one of claims 1 to 5, wherein said cationic transfecting molecule, or said nucleic acid, is combined with said stabilizing additive before combining it with said nucleic acid, or said cationic transfecting molecule, respectively.

8. The method of any one of claims 1 to 7, wherein the posiplex in the homogeneous suspension has a complex or particle size of 500 nm or less.

9. The method of any one of claims 1 to 7, wherein the posiplex in the homogeneous suspension has a complex or particle size of 300nm or less.

10. The method of any one of claims 1 to 9, wherein said cationic transfecting molecule is a cationic lipid or a cationic polymer.

11. The method of any one of claims 1 to 10, wherein the colipid is a positively or negatively charged, neutral or zwitterionic lipids.

12. The method of claim 11, wherein said colipid is phosphatidylethanolamine (PE), phosphatidylcholine, phosphocholine, dioleylphosphatidylethanolamine (DOPE), sphingomyelin, ceramide or cerebroside or one of their derivates.

13. The method of any one of claims 1 to 12, wherein the stabilizing additive is coupled to a moiety capable of being incorporated into the complex or particle.

14. The method of Claim 13, wherein the moiety is a phospholipid.

15. The method of any one of claims 1 to 14, wherein said nucleic acid component is a genomic DNA, cDNA, synthetic DNA, plasmid DNA, RNA, mRNA, ribozyme, antisense RNA or oligonucleotide.

16. The method of any one of claims 1 to 15, wherein said nucleic acid component comprises at least one gene of interest and elements which enable the said gene of interest to be expressed.

17. The method of any one of claims 1 to 16, wherein said nucleic acid component encodes all or part of a polypeptide.

18. The method of claim 17, wherein said polypeptide exhibits a therapeutic activity.

19. The method of claim 17, wherein said polypeptide exhibits a prophylactic activity, in particular an immunogenic activity.

20. The method of claim 17, wherein said polypeptide is an enzyme, a hormone, a cytokine, a membrane receptor, an antibody, a factor which regulates transcription, translation or replication and which is involved in transcript stability, a structural polypeptide, a polypeptide which forms a membrane channel, a transport polypeptide, an adhesion molecule or a ligand.

21. An homogeneous suspension of stable posiplexes obtainable by the method of any one of claims 1 to 20.

22. The homogeneous suspension of Claim 21, wherein the volume of the stabilizing additive represents from 0.05 to 99%, from 1 to 50%, or from 2 to 15%, of the total volume of said suspension.

23. A posiplex of the homogeneous suspension of claim 21 or 22 or obtainable by the method of any one of claims 1 to 20.

24. A composition comprising at least one suspension of claims 21 or 22 or at least one posiplex of claim 23, and optionally a pharmaceutically acceptable carrier.

25. Use of a suspension of claims 21 or 22 or of a posiplex of claim 23 for preparing a composition for therapeutic, prophylactic or vaccination purposes, which composition is designed for treating the human or animal body by means of gene therapy.

26. Use according to claim 25, wherein said composition is designed to be administered in a form which can be injected by the intramuscular route.

27. Use according to claim 25, wherein said composition is designed to be administered by inhalation, by intratracheal injection, by instillation or by aerosolization of the said composition.
